# EUROPÄISCHE PATENTSCHRIFT

(11) **EP 1 303 529 B1**
(45) Veröffentlichungstag und Bekanntmachung des Hinweises auf die Patenterteilung: **26.10.2005**
(21) Anmeldenummer: 00984987.8
(22) Anmeldetag: 08.11.2000
(51) Int. Cl.: C07H 21/00

(54) **VERFAHREN UND VORRICHTUNG ZUR HERSTELLUNG VON OLIGOMEREN UND ARRAYS VON OLIGOMEREN SOWIE DIE VERWENDUNG DER VORRICHTUNG**
METHOD AND DEVICE FOR PRODUCING OLIGOMERS AND ARRAYS OF OLIGOMERS AND THE USE OF SAID DEVICE
PROCEDE ET DISPOSITIF DE PRODUCTION D'OLIGOMERES ET D'ARRANGEMENTS D'OLIGOMERES ET UTILISATION DU DISPOSITIF

(30) Priorität: 10.11.1999 DE 19954009; 19.05.2000 DE 10024717
(43) Veröffentlichungstag der Anmeldung: 23.04.2003
(73) Patentinhaber: MERCKLE GMBH, 89143 Blaubeuren (DE)
(72) Erfinder: ADAMSCHIK, Mario, 89081 Ulm (DE); EGGER, Cornelia, 89129 Langenau-Hörvelsingen (DE); HINZ, Michael, 89134 Blaustein (DE); HOFER, Eberhard, P., 89173 Lonsee (DE); KOHN, Eberhard, 89081 Ulm (DE); MAIER, Claus, 89171 Illerkirchberg (DE); SELIGER, Hartmut, 89275 Elchingen-Thalfingen (DE)
(74) Vertreter: Pfenning, Meinig & Partner GbR
(86) Internationale Anmeldenummer: PCT/EP2000/011030
(87) Internationale Veröffentlichungsnummer: WO 2001/034620

(56) Entgegenhaltungen:
- EP-A- 0 035 719
- WO-A-98/36828
- WO-A-99/42805
- US-A- 4 894 664
- US-A- 5 925 732
- US-A- 5 980 704
- US-A- 5 980 719

## Beschreibung

Die Erfindung betrifft die Herstellung von Oligomeren, die durch schrittweise Kopplung von Monomereinheiten an einem festen Trägermaterial synthetisiert werden. Dabei ist der intermediäre Schutz der Monomerbausteine durch eine oder mehrere geeignete Schutzgruppen notwendig, die bei der Kopplung des Monomerbausteins auf das wachsende Kettenende übertragen werden und von denen somit eine vor dem nächsten Kopplungsschritt abgespalten werden muss, um ein reaktives (verlängerbares) Kettenende zu erzeugen. Die wachsende Kette ist dabei am Trägermaterial immobilisiert.

Üblicherweise wird das dabei verwendete Reagenz zur Abspaltung einfach vom Träger gewaschen, z.B. mit Acetonitril, jedoch konnten wir feststellen, dass ein zusätzlicher Schritt zur Neutralisation dieses Reagenzes, die Anwendung der beschriebenen Synthesemethode in manchen Fällen erleichtert oder in anderen Fällen Überhaupt erst möglich macht.

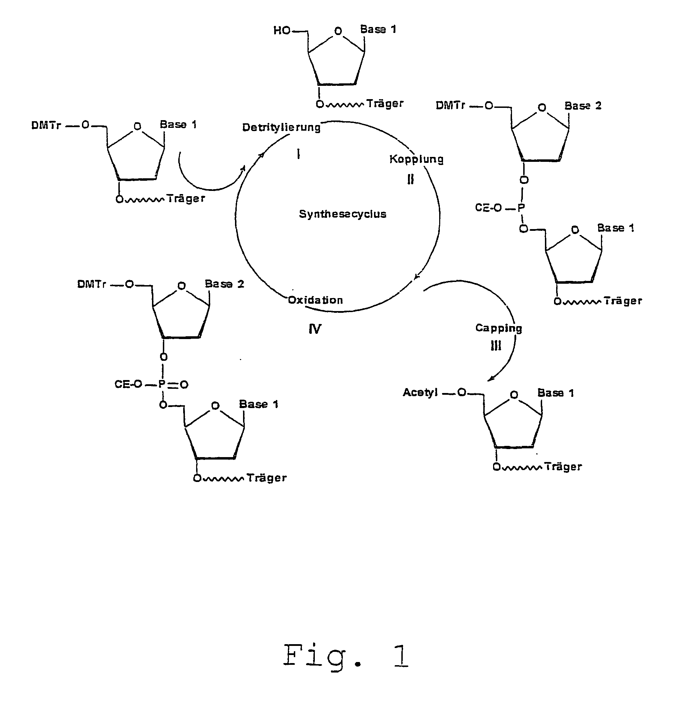

Ein Beispiel für das beschriebene Syntheseprinzip ist die Erzeugung von Oligonukleotiden nach der Methode mit Phosphorigsäure-esteramid (Amidit-Methode) oder auch der H-Phosphonat-Methode an fester Phase. Die Amidit-Methode läuft nach dem in Fig. 1 dargestellten Schema ab, bei der H-Phosphonat-Methode fehlt der Oxidationsschritt, der für alle H-Phosphonat-Einheiten am Ende der Synthese gemeinsam durchgeführt wird.

Jeder Synthesezyklus beginnt mit der Abspaltung einer Dimethoxytrityl- (bei manchen Monomeren einer Monomethoxytrityl-) Schutzgruppe ("Detritylierung"), gefolgt von der Kopplung des nächsten Monomers, einem "Cap-Schritt" zur Eliminierung nichtverlängerter Ketten und schließlich der Oxidation des Phosphors. Dieser Synthesezyklus ist weitgehend optimiert und wird auf entsprechenden Automaten routinemäßig mit Kopplungsausbeuten von über 99% pro Kopplung ausgeführt.

Dennoch ist es möglich, durch die Einführung eines einfachen zusätzlichen Schrittes in den Synthesezyklus neue Anwendungsgebiete, z.B. für die Herstellung von Oligoribonukleotiden, zu erschließen, für die der Standardzyklus sonst entweder nur mit beträchtlichem technischen Aufwand oder gar nicht einsetzbar wäre:

Die zur Abspaltung der Schutzgruppe durchgeführte Detritylierung erfolgt meist durch eine organische Säure in einem organischen Lösungsmittel, z.B. 2% Trichloressigsäure in Dichlorethan. Nach einer gewissen Zeit, z.B. 1 - 2 Minuten, wird die Säure durch Waschen entfernt, wobei oft relativ lange Waschzeiten notwendig sind, um die Säure vollständig, auch die an das Trägermaterial adsorbierte Säure, und sicher zu entfernen. Nachteilig ist das besonders im Hinblick darauf, dass diese Säure zu Depurinierungen, abhängig von der Einwirkzeit, führen kann. Ein Neutralisationsschritt dagegen beendet die Einwirkung der Säure jedoch sicher und zu einem genau definierten Zeitpunkt. Hierfür bieten sich organische Basen, wie z.B. Collidin, an.

Es ist auch bereits die Herstellung von Arrays von Oligomeren bekannt. Hierzu zählen Arrays von Oligonukleotiden, die oft auch als Oligonukleotid- oder DNA-Chips bezeichnet werden. Sie können zum einen dadurch hergestellt werden, dass zunächst die Oligonukleotide der gewünschten Sequenz hergestellt werden, die dann auf einem geeigneten Träger definiert abgelegt werden, oder indem die Synthese direkt auf dem Trägermaterial durchgeführt wird. Da sich endlich mehrere Oligomere verschiedener Sequenz auf dem Träger befinden sollen, muss die direkte Synthese auf dem Trägermaterial adressiert ablaufen, wobei entweder der ganze Synthesezyklus adressiert wird, oder aber nur der Schritt der Entschützung. Gerade die letztere Methode ist besonders reizvoll, da alle übrigen Schritte des Zyklus parallel für alle Oligomere durchgeführt werden können. Bekannt sind hierzu bisher zwei Ansätze, die aber beide erhebliche Nachteile aufweisen:
- die Verwendung von Schutzgruppen, die durch Licht abgespalten werden können (US 5 424 186)
   Nachteil dabei ist, dass vom optimierten Synthesezyklus abgewichen werden muss, dabei sinken die erreichbaren Kopplungsausbeuten spürbar, was diese Methode limitiert.
- die Verwendung von Masken, die definierte Bereiche der Synthesefläche abdecken (DE 197 06 570), oder, damit nahe verwandt, das Aufbringen der Säure durch einen Druckkopf an definierten Positionen der Synthesefläche (US 5 847 105).

Nachteil letzterer Methode ist, dass die Gefahr besteht, dass die aufgebrachte Säure verschleppt wird, also, besonders beim Abwaschen der Säure, auf Bereiche gelangt, in denen keine Synthese stattfinden soll. Aus US 5 847 105 ist der Versuch bekannt, dieses Problem zu umgehen, indem Zinkbromid statt einer Säure verwendet wird. Allerdings wird die Detritylierung dann so langsam, dass auch beim Waschen eine gewisse Chance besteht, dass Bereiche, die nicht detrityliert werden sollen, tatsächlich weitgehend unbeeinflusst bleiben. Die Synthese wird dadurch sehr zeitaufwendig, die Detritylierung mit Zinkbromid ist im Vergleich zu einer Detritylierung mit Säure (z.B. Trichloressigsäure in Dichlormethan) um wenigstens den Faktor 10 langsamer.

In DE 197 06 570 wird das Problem der Verschleppung durch die Verwendung von Masken reduziert, die auf das Synthesesubstrat (den Träger) gelegt werden und nur die Bereiche freigeben, die zu bearbeiten sind. Bei der Detritylierung kann die Säure (oder jedes andere Agens) abgewaschen werden, ohne dass die Gefahr einer Verschleppung besteht, da ja die nicht zu entschützenden Bereiche noch immer durch die Maske geschützt bleiben können. Allerdings wird die Methode eben durch die Maskentechnik auch sehr unflexibel.

Soll in einem so zu erzeugenden Array ein oder mehrere Sequenzen geändert werden, so macht das die Herstellung neuer Masken notwendig, die diese Änderungen wiederspiegeln, wodurch der arbeitstechnische wie der zeitliche Aufwand dieses Verfahrens immens ist.

Aufgabe der vorliegenden Erfindung ist es deshalb, eine verbesserte Vorrichtung mitsamt Verfahren die Herstellung von Oligomeren und Arrays von Oligomeren bereitszustellen.

Zur Lösung dieser Aufgabenstellung wird ein Verfahren vorgeschlagen, bei dem eine Verschleppung des Entschützungsreagens verhindert wird und auf einfach zu handhabende Weise mit der erfindungsgemäßen Vorrichtung eine schnellere Herstellung von Oligomeren und Arrays von Oligomeren möglich ist.

Der Neutralisationsschritt, der in dem erfindungsgemäßen Verfahren durchgeführt wird, löst diese Probleme auf einfache und elegante Weise, indem nach der nötigen Einwirkzeit der Säure auf die behandelten Positionen eine Base zur Neutralisation aufgebracht wird. Die entstehenden Produkte führen weder zu einer unerwünschten Schutzgruppenabspaltung, noch beeinträchtigen sie die weiteren Schritte des Reaktionszyklus. Die bekannten und optimierten Synthesemethoden können beibehalten werden, insbesondere sind keine neuen Monomerbausteine zu entwickeln.

Bei der hier gezeigten Methode wird eine Änderung der Hardware bei einer Änderung der herzustellenden Arrays unnötig. So erfolgt die adressierte Entschützung in einem Array von unabhängigen und gegeneinander abgeschlossenen Reaktionskammern. Diese Anordnung von Reaktionskammern spiegelt die Anordnung von Oligomeren auf dem Träger wieder, wobei die Reagenzien, die für die Abspaltung der Schutzgruppen und der Neutralisation verwendeten Reagenzien über ein Flüssigkeitsdosierelement auf definierte Positionen des Trägermaterials aufgebracht wird. Da aber die Versorgung jeder Kammer mit dem Entschützungsagens individuell gesteuert werden kann, kann dieses Array von Reaktionskammern auch jede beliebige Sequenz von Oligomeren auf dem Träger erzeugen. Wie die Versorgung der einzelnen Kammern mit Reagenzien erfolgt, ist dabei ebenso wenig von Belang, wie die Art oder der Aggregatszustand des Entschützungsagens. Das Array von Reaktionskammern kann auch als aktive Maske betrachtet werden.

Diese Methode macht aber nur dann Sinn, wenn das Entschützungsagens neutralisiert wird, um damit eine Verschleppung zu vermeiden.

Die Herstellung der Arrays von Oligomeren wird mit einer neuartigen mikroelektromechanischen Vorrichtung mit integriertem Mikroreaktor und Flüssigkeitsdosiersystem durchgeführt, wobei das Flüssigkeitsdosierelement als auch die Mikroreaktionskammer auf einem Chip integriert sind. Dabei werden über das Flüssigkeitsdosierelement die für die Abspaltung der Schutzgruppen und die Neutralisation eingesetzten Reagenzien in die Mikroreaktionskammer injiziert. Diese Flüssigkeitsdosierelemente basieren auf dem "Ink-Jet"-Prinzip und dienen zum Ausstoß bzw. der Injizierung von verschiedenen Flüssigkeiten in eine integrierte Mikroreaktionskammer.

Bei diesem Prinzip wird mit Hilfe eines Heizelementes eine sehr kleine Menge der auszustoßenden Flüssigkeit (z. B. Tinte), die sich über dem Heizer befindet, innerhalb sehr kurzer Zeit auf eine Temperatur, die zur spontanen Überhitzung notwendig ist, gebracht. Hierdurch bildet sich eine Gasblase, deren Volumenexpansion zur Ejection der verdrängten Flüssigkeitsmenge durch eine Düsenöffnung führt. Die heutzutage verwendeten "Bubble-Jet"-Systeme sind in der Regel aufgrund der Materialauswahl und vom Aufbau her auf den Druckbetrieb mit Tinte ausgelegt. Daraus folgt im wesentlichen die Einschränkung auf nicht chemisch aggressive Flüssigkeiten. Auch sind diese Systeme nicht für Anwendungen, die biokompatible Materialien voraussetzen, geeignet. In spezifischen Anwendungen in der Medizintechnik, der Biomedizintechnik, der Chemie oder auch dem Automobilbereich tritt somit das Problem auf, dass nur eine enge Auswahl an Flüssigkeiten verwendet werden könnten. Auch fehlt für viele Anwendungen wie z.B. die DNA-Sequenzierung oder Mischung von Flüssigkeiten ein geeigneter Bauelementaufbau, d.h. es fehlen integrierte Reaktionskammern, mikrofluidische Mischstrukturen usw.. Ferner können die Arrays aufgrund der hohen Oberflächenrauhigkeit der Düsenplatte nicht auf ein eventuell externes Substrat flüssigkeitsdicht aufgesetzt werden.

Mikrodosiergeräte in Verbindung mit Reaktionskammern oder Substraten, auf denen eine chemische Reaktion stattfindet, werden heute hauptsächlich in der Chemie, Medizin und in der Biochemie zur parallelen, und damit schnelleren, chemischen Synthese oder Analyse, sowie zur Mischung, Dosierung, Ejection oder Injektion verschiedenster Flüssigkeiten eingesetzt. Hierbei besteht das Gesamtsystem meistens aus einem Flüssigkeitsdosiersystem, das durch konventionelle mechanische Elemente bewegt und gesteuert wird, und einem Substrat oder Behälter, auf bzw. in dem die Reaktion stattfindet.

Beide Komponenten sind bei den vorbekannten Systemen nicht auf einem Chip integriert. Probleme, welche die meisten dieser Systeme aufbaubedingt kennzeichnen, sind die fehlende Möglichkeit der selektiv adressierbaren Flüssigkeitszuführungen, eine maßgebliche Begrenzung der Integrationsdichte, keine Dosierung von Flüssigkeitsmengen im pl-Bereich und ein daraus resultierender hoher Verbrauch an Reaktionsflüssigkeiten.

Das hier vorgestellte Konzept löst alle oben genannten Probleme mittels der Integration von mehreren Flüssigkeitsdosierelementen zusammen mit einer Reaktionskammer, beide Elemente als Einheit auch Aktuator genannt, auf einem Mikroreaktionschip. Die zur Herstellung des Chips angewendete Technologie stammt aus der Mikroelektronik und ermöglicht kleinste Bauweisen und hohe Integrationsdichten.

Der Chip besteht im einzelnen aus den Komponenten Flüssigkeitsdosier-element, Reaktionskammer und den verwendeten Substraten.
Die Aktuatoren können dabei sowohl in einem ein- oder zweidimensionalen Array angeordnet sein.

In dem hier exemplarisch hergestellten Chip sind jeweils 2 Aktuatoren über jeder Reaktionskammer angeordnet, so dass zwei unterschiedliche Flüssigkeiten ausgestoßen werden können. Allerdings können auch mehr Flüssigkeitsdosierelemente pro Reaktionskammer eingesetzt werden.

Der Chip lässt die Dosierung kleinster Flüssigkeitsmengen zu und jede Dosiereinheit ist selektiv adressierbar.

Die Flüssigkeitsdosierelemente beruhen auf einer Flüssigkeitszuführung, über die die Flüssigkeit durch ein Mikrofluidsystem in eine Düsenkammer gelangt, wo die Flüssigkeit überhitzt wird und bei der anschließenden Gasblasenexpansion durch eine Düse in die Reaktionskammer ausgestoßen wird. Die integralen Bestandteile der Flüssigkeitsdosierelemente, die Mikroheizelemente, über die die Überhitzung der Flüssigkeit erfolgt, und die Düsenplatte, sind aus chemisch inerten CVD-Diamantschichten hergestellt. Allerdings kann die Düsenplatte ebenso aus anderen inerten Materialien, wie Polyimid, Fotolack, Kunststoff, Diamant, einem Halbleiter, einem Metall oder einem Dielektrikum (SiN, SiO₂) gefertigt sein. Ebenso kann die Oberfläche des Heizelementes mit verschiedenen Materialien passiviert sein.

Die elektrische Kontaktierung der Heizelemente erfolgt hier exemplarisch mittels einer hochtemperaturstabilen Multilayermetallisierung aus Si/W:Si:N/Ti/Au. Es ist aber ebenso möglich jegliches Metall, welches einen ohm schen Kontakt zu dem Heizelement bildet, zu verwenden. Zusätzlich ist auch die Abdeckung des Metalls mit Hilfe einer Schutzschicht möglich.

Jede Reaktionskammer besitzt neben der Düsenzuführung einen zusätzlichen Kanal, der zum Druckausgleich oder der Zuführung verschiedener Flüssigkeiten oder Gase, z.B. Schutzgase, dient.

Das Mikrofluidsystem basiert auf einem aus Polyimid aufgebautem Verteilungssystem aus Kapillaren und Reservoirs, durch das die Flüssigkeiten in die Düsenkammer treten. Als Materialien kommen ebenso Fotolakke (Positivrest, Negativrest), Polymere, Metalle, Dielektrika oder Halbleiter in Frage. Dabei kann sich das mikrofluidische System sowohl auf der Vorder- als auch auf der Rückseite des mechanischen Trägersubstrates befinden.

Zu den weiteren integralen Bestandteilen des Chips gehören die verschiedenen Substrate. Hierzu zählen das mechanische Trägersubstrat, in das die Flüssigkeitszuleitungen geätzt werden und auf dessen Vorder- oder Rückseite sich das mikrofluidische System befindet, das Reaktionskammersubstrat, das die Reaktionskammer umschließt und das Reaktionsproduktesubstrat, das die Reaktionskammer von unten abschließt. Die Substrate können durch nasschemische oder trockenchemische Ätzverfahren strukturiert werden.

Als Material für diese Substrate bzw. als Oberflächenbeschichtung dieser Substrate kann Silicium, Quarz, Glas, Kunststoff, Diamant, SiC oder ein beliebiges anderes Material verwendet werden. Ebenso kann ein Mehrschichtsystem aus diesen Materialien oder einem anderen Material verwendet werden, wobei die Substrate nicht aus dem gleichen Material sein müssen. Eine weitere Möglichkeit besteht darin, sowohl das Reaktionskammersubstrat als auch das Reaktionsproduktesubstrat durch das zuvor beschriebene mikrofluidische System zu ersetzten.

Die chemische Reaktion findet auf dem Reaktionsproduktesubstrat statt, das in dem hier hergestellten Chip nicht mechanisch fest verbunden, sondern abnehmbar ist. Auf dem Reaktionsproduktesubstrat findet in der hier genutzten Applikation die Synthese der Oligonukleotidketten statt. Die Oberfläche des Reaktionsproduktesubstrats ist chemisch funktionalisiert. Ebenso können die Düsenplatte und das Reaktionskammersubstrat funktionalisiert sein. Die funktionalisierten Bereiche sind ferner strukturiert. Nach der Synthese kann das Reaktionsproduktesubstrat abgenommen werden und weiter genutzt werden.

Eine weitere Ausgestaltung der Erfindung sieht vor, dass das Reaktionsproduktesubstrat auch transparent sein kann. Dies ermöglicht den Einsatz analytischer Geräte mit der erfindungsgemäßen Vorrichtung, wie z.B. Mikroskopen, CCD-Kameras, Photodioden und Phototransistoren. Zusätzlich ist auch die direkte Integrierung elektronischer Bauelemente wie Transistoren, Dioden, CCD's, Fotodioden, Fototransistoren, Widerständen oder Elektroden zur Impedanzmessung oder für zyklische Voltammetrie möglich.

Nachfolgend wird die Erfindung anhand von Figuren und bevorzugten Ausführungsbeispielen näher beschrieben. Es zeigen:
- Fig. 1:: Synthesezyklus der Amidit-Methode mit Phosphorigsäure-esteramid;
- Fig. 2:: die Strukturierung einer erfindungsgemäßen mikroelektromechanischen Vorrichtung;
- Fig. 3:: die Darstellung für die Gleichgewichtsreaktion der Detritylierung im sauren Medium;
- Fig. 4:: die Strukturierung des Synthesemoduls für die Kopplung, das Capping und die Oxidation und des Synthesemoduls für die adressierte Entschützung

In Figur 1 ist das Schema des Synthesezyklus der Amidit-Methode mit Phosphorigsäure-esteramid für die Herstellung von Oligonukleotiden dargestellt.

Der Zyklus beruht in Schritt I auf der Abspaltung einer Di- bzw. Monomethoxytrityl-Schutzgruppe. Dieser Schritt wird als Detritylierung bezeichnet. In Schritt II erfolgt die Kopplung des Monomers an der ungeschützten Position. Schritt III, genannt "Capping" sorgt für eine Elimination nichtverlängerter Ketten. Im abschließenden Schritt IV erfolgt schließlich die Oxidation des Phosphors.

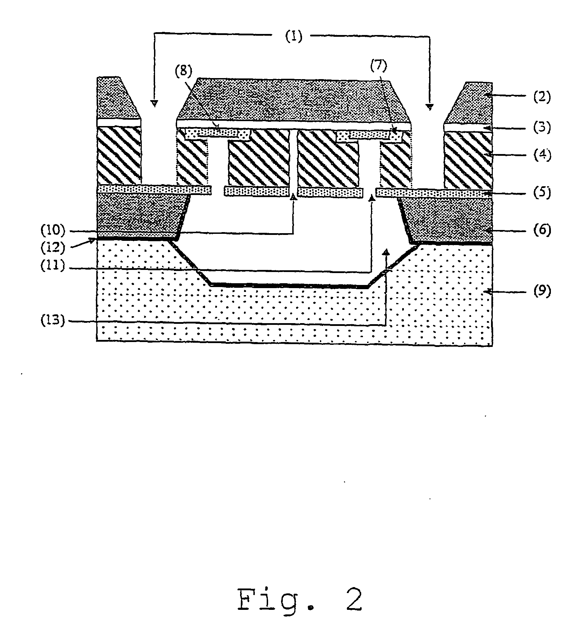

In Figur 2 ist die Strukturierung der erfindungsgemäßen mikroelektromechanischen Vorrichtung dargestellt.

Der hier vorgestellte Chip löst die oben genannten Probleme und stellt ein integriertes System, bestehend aus mehreren Flüssigkeitsdosierelementen (1), (4), (8), (7), (5), einem mikrofluidischen System (4) und einer Reaktionskammer (13), dar. Neben dem neuartigen Aufbau wurden auch neuartige Materialien wie CVD-Diamantschichten für die Mikroheizelemente (8), die Düsenplatte (5) und die Beschichtung des Reaktionskammersubstrats (6) und des Reaktionsproduktesubstrats (9) benutzt. Die Diamantschicht für die Heizelemente (8) wurde in situ bordotiert und trokkenchemisch (Ar/0₂-Plasma) strukturiert. Die Dotierstoffkonzentration betrug ca. 10²⁰ cm⁻³. Damit wurde ein spezifischer Widerstand im mΩcm Bereich erreicht. Ferner wurde eine undotierte CVD-Diamantschicht auf Si als Düsenplatte (5) und als Beschichtung (12) eingesetzt. Die Düsen (11) wurden ebenfalls trockenchemisch geätzt. Diamant besitzt hervorragende mechanische, chemische und thermische Eigenschaften. Er ist chemisch völlig inert und mechanisch sehr stabil, weshalb alle nasschemischen Substanzen eingesetzt werden können. Es sind keine Passivierungsschichten gegen Oxidation, Kavitationsschäden oder gegen einen chemischen Angriff des Mikroheizers und auch der Düsenplatte notwendig. Durch die geringe Oberflächenrauhigkeit können Standardlithogrpahieprozesse aus der Mikroelektronik zur Strukturierung verwendet werden.

Das mechanische Trägersubstrat (2) besteht aus Silizium in das nasschemisch die Zuführungen (1) für zwei unterschiedliche Flüssigkeiten geätzt wurden. Das mechanische Trägersubstrat (2) ist mit einer dünnen (2µm) Schicht SiO₂ (3), die als thermischer Isolator für die Mikroheizelemente (8) dient, beschichtet. Die Flüssigkeiten werden mit Hilfe eines aus Polyimid aufgebauten mikrofluidischen Verteilungssystem aus Kapillaren und Reservoirs (4) von der Rückseitenzuführung (1) auf die jeweiligen Diamantheizelemente (8) geleitet. Die Mikroheizelemente (8) dienen zur Überhitzung der verwendeten Flüssigkeit die infolge der Gasblasenvolumenexpansion durch die Diamantdüsenplatte (5) und der Düse (11) in die Mikroreaktionskammer (13) ausgestoßen wird. Die Heizelemente werden mittels einer hochtemperaturstabilen Multilayermetallisierung aus Si/W:Si:N/Ti/Au (7) elektrisch kontaktiert. Die chemische Reaktion findet auf dem Reaktionsproduktesubstrat (9) statt, das in dem hier hergestellten Chip nicht mechanisch fest verbunden, sondern abnehmbar ist. Auf dem Reaktionsproduktesubstrat (9) findet in der hier genutzten Applikation die Synthese der Oligonukleotidketten statt. Die Oberfläche des Reaktionsproduktesubstrats (9) ist chemisch funktionalisiert. Die funktionalisierten Bereiche sind ferner strukturiert. Nach der Synthese kann das Substrat abgenommen werden und weiter genutzt werden. Jede Reaktionskammer (13) enthält einen Kanal (10), welcher zum Druckausgleich oder zur Zuführung verschiedener Flüssigkeiten oder Gase, z.B. Schutzgase dient.

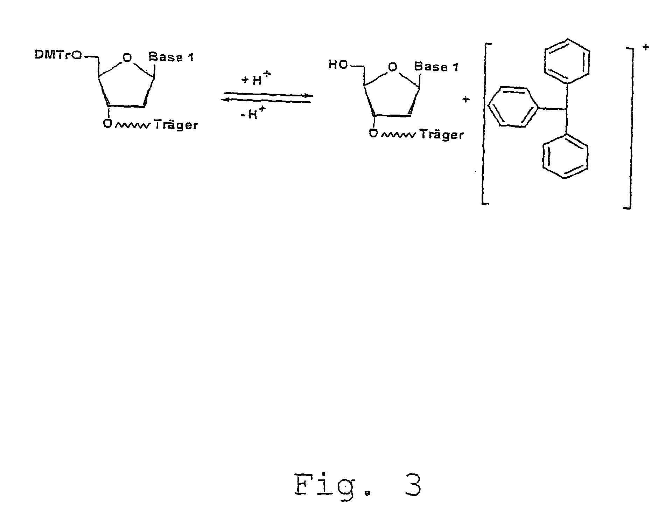

In Figur 3 ist das Gleichgewicht der Detritylierungsreaktion eines immobilisierten Oligonukleotids dargestellt. Die Hinreaktion, d.h. die Detritylierung zum entschützten Oligonukleotid erfolgt dabei durch Zugabe einer Säure. Bei der Neutralisation, z.B. mit Collidin, bilden sich Reaktionsprodukte, die die Synthese nicht beeinträchtigen. Die Rückreaktion fällt daher kaum ins Gewicht.

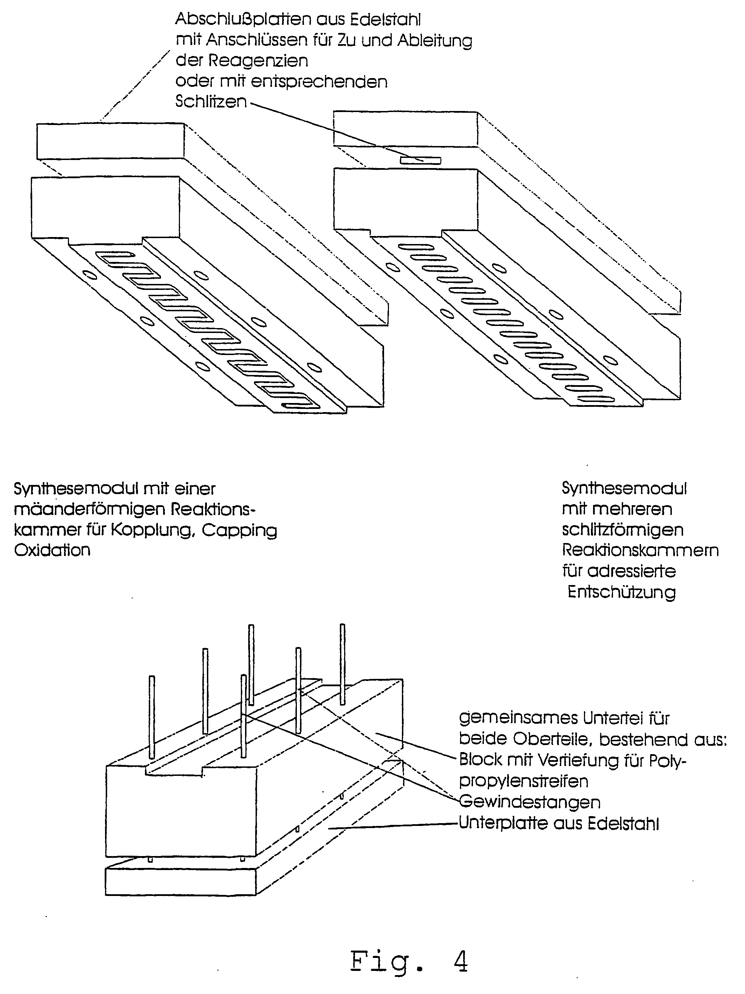

In Figur 4 ist der Aufbau der Synthesekammern dargestellt. Die Synthesekammer besteht aus einem Unterteil, auf das die Folie als Reaktionsproduktesubstrat, die in einem Rahmen eingespannt ist, aufgelegt wird, und zwei Oberteilen, von denen eines die adressierte Entschützung / Neutralisation ermöglicht, das zweite die Durchführung aller übrigen Schritte des Zyklus für die gesamte Folie.

Das Oberteil für die Entschützung/Neutralisation ist im Wesentlichen eine Platte mit mehreren schlitzförmigen Reaktionskammern, die mit mehreren Flüssigkeitsdosierelementen bestückt ist. Für die adressierte Entschützung wird an den zu entschützenden Positionen zunächst die Säure, die eine örtlich definierte Detritylierung bewirkt, dann die Base eingebracht. Beides geschieht manuell.

Die zweite Platte beinhaltet eine Synthesekammer, die alle möglichen Positionen abdeckt und gemeinsam mit Reagenzien versorgt. Für jede Kettenverlängerung muss, ebenfalls manuell, einmal zwischen den Kammern gewechselt werden.

### Beispiel 1:

Die Sequenz .dA₂₀ wurde auf einem Pharmacia Gene Assembler dergestalt erzeugt, dass der Detritylierungsschritt aus dem Programm entfernt wurde und die Synthesekartusche mit dem Trägermaterial außerhalb des Geräts mit 0.5 mL 2% Trichloressigsäure in Dichlorethan versetzt wurde. Nach 1 Minute wurde mit 0.5 mL 10% Collidin in Acetonitril neutralisiert, die Kartusche nach gründlicher Durchmischung aus der Lösung entnommen, noch nass wieder in das Gerät eingesetzt und der Reaktionszyklus fortgeführt. Die Analyse des so erzeugten Oligonukleotids zeigt, dass die Reaktionsprodukte der Neutralisation die Synthese nicht beeinträchtigen und die mögliche Rückreaktion (Fig. 3) nicht merklich ins Gewicht fällt.

### Beispiel 2:

### Manuelle Synthese eines zweidimensionalen Oligonukleotid-Arrays.

Die Synthese wurde in einer speziellen Synthesekammer, die an kommerziell erhältliche Synthesizer angeschlossen werden kann, auf einer funktionalisierten polypropylenfolie⁴, durchgeführt. Die Synthesekammer besteht aus einem Unterteil, auf das die Folie, die in einem Rahmen eingespannt ist, aufgelegt wird, und zwei Oberteilen, von denen eines die adressierte Entschützung / Neutralisation ermöglicht, das zweite die Durchführung aller übrigen Schritte des Zyklus für die gesamte Folie. Die Vorrichtung ist auch für die Verwendung anderer Trägermaterialien geeignet.
Das Oberteil für die Entschützung/Neutralisation ist im Wesentlichen eine gelochte Platte; in die Öffnungen wird an den zu entschützenden Positionen zunächst die Säure, die eine örtlich definierte Detritylierung bewirkt, dann die Base eingebracht. Beides geschieht manuell. Die zweite Platte beinhaltet eine Synthesekammer, die alle möglichen Positionen abdeckt und gemeinsam mit Reagenzien versorgt. Für jede Kettenverlängerung muss, ebenfalls manuell, einmal zwischen den Kammern gewechselt werden.
Vor dem ersten adressierten Synthesezyklus wurde in der Kammer II auf die Folie eine Kopplung mit einen C₆-Aminolink durchgeführt, dadurch können die erzeugten Sequenzen auf Wunsch für eine Qualitätskontrolle abgespalten werden.

### Prinzipielle Vorgehensweise bei jeder Kettenverlängerung:

1. Oberteil I wird aufgesetzt
2. Säure wird in die Öffnungen, die den zu verlängernden Positionen entsprechen, eingebracht.
3. Warten bis zur vollständigen Detritylierung (30 sec.)
4. Base wird in die mit Säure beaufschlagten Öffnungen eingebracht
5. (optional) Entfernen der Flüssigkeit aus den betreffenden Öffnungen
6. Kammer I wird entfernt
7. Kammer II wird aufgesetzt und an den Synthesizer angeschlossen
8. der Synthesizer führt die Schritte "Kopplung eines Monomers", "Capping" und "Oxidation" nach Vorschrift des Geräteherstellers durch.

### Beispiel 3:

### Manuelle Synthese eines eindimensionalen Oligonukleotid-Arrays.

Die Vorgehensweise entspricht der aus Beispiel 2. Die Synthese wurde in einer speziellen Synthesekammer, die an kommerziell erhältliche Synthesizer angeschlossen werden kann, auf einem funktionalisierten Polypropylenstreifen, durchgeführt. Die Synthesekammer besteht aus einem Unterteil, auf das der Streifen aufgelegt wird, und zwei Oberteilen, von denen eines die adressierte Entschützung / Neutralisation, das zweite die Durchführung aller übrigen Schritte des Zyklus für den gesamten Streifen ermöglicht. Die Vorrichtung ist auch für die Verwendung anderer Trägermaterialien geeignet.
Das Oberteil für die Entschützung/Neutralisation ist im Wesentlichen eine mit Schlitzen versehene Platte, in die Schlitze wird an den zu entschützenden Positionen zunächst die Säure, die eine örtlich definierte Detritylierung bewirkt, dann die Base eingebracht. Beides geschieht manuell. Die zweite Platte beinhaltet eine mäanderförmige Synthesekammer, die alle möglichen entschützten Positionen abdeckt und gemeinsam mit Reagenzien versorgt (Fig. 4). Für jede Kettenverlängerung muss, ebenfalls manuell, einmal zwischen den Kammern gewechselt werden
Vor dem ersten adressierten Synthesezyklus wurde in der Kammer II auf die Folie eine Kopplung mit einen C6-Aminolink durchgeführt, dadurch können die erzeugten Sequenzen auf Wunsch für eine Qualitätskontrolle abgespalten werden.

## Patentansprüche

1. Mikroelektromechanische Vorrichtung zur Herstellung von Oligomeren,
**dadurch gekennzeichnet, dass** mindestens ein Flüssigkeitsdosierelement (1, 4, 5, 7, 8) mit mindestens einer Mikroreaktionskammer (13) auf einem Chip integriert ist, wobei das Flüssigkeitsdosierelement ein Mikroheizelement (8) und einen in eine Düse (11) mündenden Kanal (10) zum Austreiben der Flüssigkeit aufweist und die Mikroreaktionskammer (13) sich unmittelbar an die Düse (11) auf deren dem Kanal (10) abgewandten Seite anschließt.

2. Vorrichtung nach Anspruch 1,
**dadurch gekennzeichnet, dass** mehrere Reaktionskammern (13) als ein- oder mehrdimensionales Array angeordnet sind.

3. Vorrichtung nach mindestens einem der Ansprüche 1 oder 2, **dadurch gekennzeichnet, dass** jede Reaktionskammer (13) mindestens zwei Flüssigkeitsdosierelement (1, 4, 5, 7, 8) besitzt.

4. Vorrichtung nach mindestens einem der Ansprüche 1 bis 3, **dadurch gekennzeichnet, dass** jedes Flüssigkeitsdosierelement (1, 4, 5, 7, 8) selektiv adressierbar ist.

5. Vorrichtung nach mindestens einem der Ansprüche 1 bis 4,
**dadurch gekennzeichnet, dass** durch Überhitzung einer Flüssigkeit und einer anschließenden Gasblasenexplosion die Flüssigkeit durch die Düse (11) in eine Reaktionskammer (13) ausgestoßen wird.

6. Vorrichtung nach mindestens einem der Ansprüche 1 bis 5, **dadurch gekennzeichnet, dass** die Mikroheizelemente (8) und die Düsenplatte (5) aus chemisch inerten Materialien hergestellt sind.

7. Vorrichtung nach mindestens einem der Ansprüche 1 bis 6,
**dadurch gekennzeichnet, dass** die Düsenplatte (5) auch aus Polyimid, Fotolack, Kunststoff, Diamant, einem Halbleiter, einem Metall oder einem Dielektrikum (SiN, SiO₂) besteht.

8. Vorrichtung nach mindestens einem der Ansprüche 1 bis 7,
**dadurch gekennzeichnet, dass** das Mikroheizelement (8) an der Oberfläche mit verschiedenen Materialien passiviert ist.

9. Vorrichtung nach mindestens einem der Ansprüche 1 bis 8,
**dadurch gekennzeichnet, dass** für die Metallisierung (7) ein beliebiges Metall, welches einen Ohm'schen Kontakt zu dem Mikroheizelement (8) bildet, verwendet wird.

10. Vorrichtung nach mindestens einem der Ansprüche 1 bis 9,
**dadurch gekennzeichnet, dass** die Metallisierung (7) durch eine Schutzschicht abgedeckt ist.

11. Vorrichtung nach mindestens einem der Ansprüche 1 bis 10,
**dadurch gekennzeichnet, dass** jede Reaktionskammer (13) einen Kanal (10) zur Entlüftung oder zur Zufuhr zusätzlicher Flüssigkeiten oder Schutzgase enthält.

12. Vorrichtung nach mindestens einem der Ansprüche 1 bis 11,
**dadurch gekennzeichnet, dass** das mikrofluidische System (4) auch aus einem Fotolack (Positivrest, Negativrest), einem Polymer, einem Polyimid, einem Metall, einem Dielektrikum oder einem Halbleiter besteht.

13. Vorrichtung nach mindestens einem der Ansprüche 1 bis 12,
**dadurch gekennzeichnet, dass** die Substrate (2, 6, 9) mit Hilfe von nasschemischen oder trockenchemischen Ätzverfahren strukturiert sind.

14. Vorrichtung nach mindestens einem der Ansprüche 1 bis 13,
**dadurch gekennzeichnet, dass** sich das mikrofluidische System (4) für die Reaktionsflüssigkeiten auf der Vorderseite oder der Rückseite des mechanischen Trägersubstrates (2) befindet.

15. Vorrichtung nach mindestens einem der Ansprüche 1 bis 14,
**dadurch gekennzeichnet, dass** die Substrate (2, 6, 9) aus Silicium, Quarz, Glas, Kunststoff, Diamant, SiC oder einem beliebigen anderen Material oder einem Mehrschichtsystem aus den genannten oder einem anderen Material besteht.

16. Vorrichtung nach mindestens einem der Ansprüche 1 bis 15,
**dadurch gekennzeichnet, dass** das Reaktionskammersubstrat (6) komplett oder nur teilweise mit Diamant, SiC, SiO₂, Si₃N₄, Metallen, Kunststoffen oder Dielektrika beschichtet wird.

17. Vorrichtung nach mindestens einem der Ansprüche 1 bis 16,
**dadurch gekennzeichnet, dass** das Reaktionsproduktesubstrat (9) komplett oder nur teilweise mit Diamant, SiC, SiO₂, Si₃N₄, Metallen, Kunststoffen oder Dieleketrika beschichtet wird.

18. Vorrichtung nach mindestens einem der Ansprüche 1 bis 17,
**dadurch gekennzeichnet, dass** die Substrate (6, 9) durch ein mikrofluidisches System analog (4) ersetzt werden.

19. Vorrichtung nach mindestens einem der Ansprüche 1 bis 18,
**dadurch gekennzeichnet, dass** die Flüssigkeitsdosierelemente enthaltende Teil der Vorrichtung und der die Reaktionskammer enthaltende Teil der Vorrichtung voneinander lösbar ausgebildet sind.

20. Vorrichtung nach mindestens einem der Ansprüche 1 bis 19,
**dadurch gekennzeichnet, dass** das Reaktionsproduktesubstrat (9) zur Mischung, Synthese oder einer beliebigen chemischen Reaktion verwendet wird.

21. Vorrichtung nach mindestens einem der Ansprüche 1 bis 20,
**dadurch gekennzeichnet, dass** das Reaktionsproduktesubstrat (9), die Düsenplatte (5) oder das Reaktionskammersubstrat (6) chemisch oberflächenbehandelt (z.B. funktionalisiert oder terminiert) wird.

22. Vorrichtung nach mindestens einem der Ansprüche 1 bis 21,
**dadurch gekennzeichnet, dass** das Reaktionsproduktesubstrat (9) transparent ist, um darunter Analysengeräte wie z.B. Mikroskope, CCD-Kameras, Photodioden, Phototransistoren einzusetzen.

23. Vorrichtung nach mindestens einem der Ansprüche 1 bis 22,
**dadurch gekennzeichnet, dass** im Reaktionsproduktesubstrat (9) elektronische Bauelemente wie Transistoren, Dioden, CCD's, Fotodioden, Fototransistoren, Widerstände oder Elektroden zur Impedanzmessung oder für zyklische Voltammetrie integriert sind.

24. Vorrichtung nach mindestens einem der Ansprüche 1 bis 23,
**dadurch gekennzeichnet, dass** in die Reaktionskammer (13) oder auf die Substrate (6, 9) elektronische Bauelemente wie Transistoren, Dioden, CCD's, Fotodioden, Fototransistoren, Widerstände oder Elektroden zur Impedanzmessung oder für die zyklische Voltammetrie integriert sind.

25. Vorrichtung nach mindestens einem der Ansprüche 1 bis 24,
**dadurch gekennzeichnet, dass** die Reaktionskammer (13) als Vertiefung in einem Substrat (6, 9) ausgebildet ist.

26. Verfahren zur Herstellung von Oligomeren oder Arrays von Oligomeren unter Verwendung der mikroelektromechanischen Vorrichtung nach einem der Ansprüche 1 bis 25 durch schrittweise Kopplung von Monomereinheiten an ein festes Trägermaterial, wobei der intermediäre Schutz durch eine oder mehrere Schutzgruppen erfolgt, die bei der Kopplung der Monomereinheit auf das wachsende Kettenende übertragen werden und vor dem nächsten Kopplungsschritt durch Schutzgruppenabspaltung entfernt werden, und nach der Abspaltung der Schutzgruppe ein zusätzlicher Neutralisationsschritt durchgeführt wird, wobei die zur Abspaltung der Schutzgruppe und zur Neutralisation verwendeten Reagenzien in gegeneinander abgeschlossenen Reaktionskammern (13) eingebracht werden.

27. Verfahren nach Anspruch 26,
**dadurch gekennzeichnet, dass** als Oligomere Oligonukleotide eingesetzt werden.

28. Verfahren nach Anspruch 26,
**dadurch gekennzeichnet, dass** als Oligomere Oligoribonukleotide eingesetzt werden.

29. Verfahren nach mindestens einem der Ansprüche 26 bis 28,
**dadurch gekennzeichnet, dass** abiologische Monomereinheiten Verwendung finden.

30. Verfahren nach mindestens einem der Ansprüche 26 bis 29,
**dadurch gekennzeichnet, dass** die Abspaltung der Schutzgruppe durch eine Säure, z.B. Trichloressigsäure, erfolgt.

31. Verfahren nach mindestens einem der Ansprüche 26 bis 30,
**dadurch gekennzeichnet, dass** die zur Neutralisation verwendete Base eine organische Base, z.B. Collidin ist.

32. Verfahren nach mindestens einem der Ansprüche 26 bis 31,
**dadurch gekennzeichnet, dass** die Injektion der Reagenzien für die Abspaltung der Schutzgruppen und die Neutralisation über ein Flüssigkeitsdosierelement (1, 4, 5, 7, 8) in eine Mikroreaktionskammer (13) erfolgt, wobei das Flüssigkeitsdosierelement und die Mikroreaktionskammer auf einem Chip integriert sind.

33. Verfahren nach mindestens einem der Ansprüche 26 bis 32,
**dadurch gekennzeichnet, dass** die zur Abspaltung der Schutzgruppe und zur Neutralisation verwendeten Reagenzien durch ein Flüssigkeitsdosierelement (1, 4, 5, 7, 8) auf definierte Positionen des Trägermaterials aufgebracht werden.

34. Verwendung der Vorrichtung nach mindestens einem der Ansprüche 1 bis 25, zur Analyse von DNA-Ketten oder Oligomeren.

35. Verwendung der Vorrichtung nach mindestens einem der Ansprüche 1 bis 25 für die Mischung und Reaktion verschiedener, auch aggressiver Flüssigkeiten.

36. Verwendung der Vorrichtung nach mindestens einem der Ansprüche 1 bis 25 zur Dosierung und/oder Mischung verschiedener Flüssigkeiten auf einem Feststoff, z.B. zur Analyse oder Reaktion des Feststoffs.

## Claims

1. Microelectromechanical device for producing oligomers,
**characterized in that** at least one liquid metering element (1, 4, 5, 7, 8) is integrated with at least one microreaction chamber (13) on a chip, the liquid metering element having a microheating element (8) and a channel (10) opening into a nozzle (11) and serving for expelling the liquid, and the microreaction chamber (13) directly adjoining the nozzle (11) on that side of the latter which is remote from the channel (10).

2. Device according to Claim 1,
**characterized in that** a plurality of reaction chambers (13) are arranged as a one- or multidimensional array.

3. Device according to at least one of Claims 1 or 2,
**characterized in that** each reaction chamber (13) has at least two liquid metering elements (1, 4, 5, 7, 8).

4. Device according to at least one of Claims 1 to 3,
**characterized in that** each liquid metering element (1, 4, 5, 7, 8) is selectively addressable.

5. Device according to at least one of Claims 1 to 4,
**characterized in that**, as a result of overheating of a liquid and a subsequent gas bubble explosion, the liquid is ejected through the nozzle (11) into a reaction chamber (13).

6. Device according to at least one of Claims 1 to 5,
**characterized in that** the microheating elements (8) and the nozzle plate (5) are produced from chemically inert materials.

7. Device according to at least one of Claims 1 to 6,
**characterized in that** the nozzle plate (5) also comprises polyimide, photoresist, plastic, diamond, a semiconductor, a metal or a dielectric (SiN, SiO₂).

8. Device according to at least one of Claims 1 to 7,
**characterized in that** the microheating element (8) is passivated with different materials at the surface.

9. Device according to at least one of Claims 1 to 8,
**characterized in that** an arbitrary metal which forms an ohmic contact to the microheating element (8) is used for the metallization (7).

10. Device according to at least one of Claims 1 to 9,
**characterized in that** the metallization (7) is covered by a protective layer.

11. Device according to at least one of Claims 1 to 10,
**characterized in that** each reaction chamber (13) contains a channel (10) for venting or for feeding of additional liquids or protective gases.

12. Device according to at least one of Claims 1 to 11,
**characterized in that** the microfluidic system (4) also comprises a photoresist (positive residue, negative residue), a polymer, a polyimide, a metal, a dielectric or a semiconductor.

13. Device according to at least one of Claims 1 to 12,
**characterized in that** the substrates (2, 6, 9) are patterned with the aid of wet-chemical or dry-chemical etching methods.

14. Device according to at least one of Claims 1 to 13,
**characterized in that** the microfluidic system (4) for the reaction liquids is situated on the front side or the rear side of the mechanical carrier substrate (2).

15. Device according to at least one of Claims 1 to 14,
**characterized in that** the substrates (2, 6, 9) comprise silicon, quartz, glass, plastic, diamond, SiC or an arbitrary other material or a multilayer system comprising the aforementioned materials or some other material.

16. Device according to at least one of Claims 1 to 15,
**characterized in that** the reaction chamber substrate (6) is completely or only partially coated with diamond, SiC, SiO₂, Si₃N₄, metals, plastics or dielectrics.

17. Device according to at least one of Claims 1 to 16,
**characterized in that** the reaction product substrate (9) is completely or only partially coated with diamond, SiC, SiO₂, Si₃N₄, metals, plastics or dielectrics.

18. Device according to at least one of Claims 1 to 17,
**characterized in that** the substrates (6, 9) are replaced analogously (4) by a microfluidic system.

19. Device according to at least one of Claims 1 to 18,
**characterized in that** the part of the device which contains the liquid metering elements and the part of the device which contains the reaction chamber are formed such that they can be released from one another.

20. Device according to at least one of Claims 1 to 19,
**characterized in that** the reaction product substrate (9) is used for mixing, synthesis or an arbitrary chemical reaction.

21. Device according to at least one of Claims 1 to 20,
**characterized in that** the reaction product substrate (9), the nozzle plate (5) or the reaction chamber substrate (6) is chemically surface-treated (e.g. functionalized or terminated).

22. Device according to at least one of Claims 1 to 21,
**characterized in that** the reaction product substrate (9) is transparent in order to insert underneath analysis apparatuses such as e.g. microscopes, CCD cameras, photodiodes, phototransistors.

23. Device according to at least one of Claims 1 to 22,
**characterized in that** electronic components such as transistors, diodes, CCDs, photodiodes, phototransistors, resistors or electrodes for impedance measurement or for cyclic voltametry are integrated in the reaction product substrate (9).

24. Device according to at least one of Claims 1 to 23,
**characterized in that** electronic components such as transistors, diodes, CCDs, photodiodes, phototransistors, resistors or electrodes for impedance measurement or for cyclic voltametry are integrated into the reaction chamber (13) or onto the substrates (6, 9).

25. Device according to at least one of Claims 1 to 24,
**characterized in that** the reaction chamber (13) is formed as a depression in a substrate (6, 9).

26. Method for producing oligomers or arrays of oligomers using the microelectromechanical device according to one of Claims 1 to 25 by step-by-step coupling of monomer units to a solid carrier material, the intermediate protection being effected by one or more protective groups which are transferred to the growing chain end during the coupling of the monomer unit and are removed by protective group elimination prior to the next coupling step, and an additional neutralization step being carried out after the elimination of the protective group, the reagents used for the elimination of the protective group and for the neutralization being introduced in reaction chambers (13) that are closed off from one another.

27. Method according to Claim 26,
**characterized in that** oligonucleotides are used as oligomers.

28. Method according to Claim 26,
**characterized in that** oligoribonucleotides are used as oligomers.

29. Method according to at least one of Claims 26 to 28,
**characterized in that** abiological monomer units are used.

30. Method according to at least one of Claims 26 to 29,
**characterized in that** the protective group is eliminated by means of an acid, e.g. trichloroacetic acid.

31. Method according to at least one of Claims 26 to 30,
**characterized in that** the base used for the neutralization is an organic base, e.g. collidine.

32. Method according to at least one of Claims 26 to 31,
**characterized in that** the injection of the reagents for the elimination of the protective groups and the neutralization is effected by means of a liquid metering element (1, 4, 5, 7, 8) into a microreaction chamber (13), the liquid metering element and the microreaction chamber being integrated on a chip.

33. Method according to at least one of Claims 26 to 32,
**characterized in that** the reagents used for the elimination of the protective group and for the neutralization are applied to defined positions of the carrier material by means of a liquid metering element (1, 4, 5, 7, 8).

34. Use of the device according to at least one of Claims 1 to 25 for the analysis of DNA chains or oligomers.

35. Use of the device according to at least one of Claims 1 to 25 for the mixing and reaction of different, including aggressive, liquids.

36. Use of the device according to at least one of Claims 1 to 25 for the metering and/or mixing of different liquids on a solid, e.g. for analysis or reaction of the solid.

## Revendications

1. Dispositif microélectromécanique pour la fabrication d'oligomères,
**caractérisé en ce qu'**au moins un élément de dosage de liquide (1, 4, 5, 7, 8) avec au moins une chambre de microcréation (13) est intégré sur une puce, l'élément de dosage de liquide présentant un microélément de chauffage (8) et un canal (10) débouchant dans une buse (11) pour l'évacuation du liquide, la microchambre de réaction (13) faisant suite directement à la buse (11) sur son côté opposé au canal (10).

2. Dispositif selon la revendication 1,
**caractérisé en ce que en ce que** plusieurs chambres de réaction (13) sont disposées sous la forme d'un réseau à une ou plusieurs dimensions.

3. Dispositif selon au moins l'une quelconque des revendications 1 ou 2, **caractérisé en ce que** chaque chambre de réaction (13) présente au moins deux éléments de dosage de liquide (1, 4, 5, 7, 8).

4. Dispositif selon au moins l'une quelconque des revendications 1 à 3, **caractérisé en ce que** chaque élément de dosage de liquide (1, 4, 5, 7, 8) peut être adressé de façon sélective.

5. Dispositif selon au moins l'une quelconque des revendications 1 à 4,
**caractérisé en ce que**, par la surchauffe d'un liquide et d'une explosion consécutive de bulles de gaz, le liquide est expulsé à travers la buse (11) dans une chambre de réaction (13).

6. Dispositif selon au moins l'une quelconque des revendications 1 à 5, **caractérisé en ce que** les microéléments de chauffage (8) et la plaque de buse (5) sont fabriqués à partir de matériaux chimiquement inertes.

7. Dispositif selon au moins l'une quelconque des revendications 1 à 6,
**caractérisé en ce que** la plaque de buse (5) est également à base de polyimide, de photorésist, de plastique, de diamant, d'un semi-conducteur, d'un métal ou d'un diélectrique (SiN, SiO₂).

8. Dispositif selon au moins l'une quelconque des revendications 1 à 7
**caractérisé en ce que** le microélément de chauffage (8) est passivé sur la surface avec différents matériaux.

9. Dispositif selon au moins l'une quelconque des revendications 1 à 8,
**caractérisé en ce que**, pour la métallisation (7), on utilise un métal quelconque qui forme un contact ohmique avec le microélément de chauffage (8).

10. Dispositif selon au moins l'une quelconque des revendications 1 à 9,
**caractérisé en ce que** la métallisation (7) est recouverte par une couche de protection.

11. Dispositif selon au moins l'une quelconque des revendications 1 à 10,
**caractérisé en ce que** chaque chambre de réaction (13) contient un canal (10) pour la ventilation ou l'arrivée de liquides ou de gaz de protection supplémentaires.

12. Dispositif selon au moins l'une quelconque des revendications 1 à 11,
**caractérisé en ce que** le système (4) microfluidique se compose également d'un photorésist (reste positif, reste négatif), d'un polymère, d'un polyimide, d'un métal, d'un diélectrique ou d'un semi-conducteur.

13. Dispositif selon au moins l'une quelconque des revendications 1 à 12,
**caractérisé en ce que** les substrats (2, 6, 9) sont structurés à l'aide de procédés de décapage à chimie humide ou à chimie sèche.

14. Dispositif selon au moins l'une quelconque des revendications 1 à 13,
**caractérisé en ce que** le système microfluidique (4) pour les liquides de réaction se trouve sur le côté avant ou sur le côté arrière du substrat porteur (2) mécanique.

15. Dispositif selon au moins l'une quelconque des revendications 1 à 14,
**caractérisé en ce que** les substrats (2, 6, 9) sont à base de silicium, quartz, verre, plastique, diamant, SiC ou un autre matériau quelconque ou un système multicouche à base des matériaux cités ou d'un autre matériau.

16. Dispositif selon au moins l'une quelconque des revendications 1 à 15,
**caractérisé en ce que** le substrat de la chambre de réaction (6) est recouvert complètement ou seulement partiellement avec du diamant, du SiC, du SiO₂, du Si₃N₄, des métaux, des plastiques ou des diélectriques.

17. Dispositif selon au moins l'une quelconque des revendications 1 à 16,
**caractérisé en ce que** le substrat de produits de réaction (9) est recouvert complètement ou seulement partiellement avec du diamant, du SiC, du SiO₂, du Si₃N₄, des métaux, des plastiques ou des diélectriques.

18. Dispositif selon au moins l'une quelconque des revendications 1 à 17,
**caractérisé en ce que** les substrats (6, 9) sont remplacés de façon analogue (4) par un système microfluidique.

19. Dispositif selon au moins l'une quelconque des revendications 1 à 18,
**caractérisé en ce que** la partie, contenant les éléments de dosage de liquide, du dispositif et la partie, contenant la chambre de réaction, du dispositif sont réalisées de façon détachable l'une de l'autre.

20. Dispositif selon au moins l'une quelconque des revendications 1 à 19,
**caractérisé en ce que** le substrat de produits de réaction (9) est utilisé pour le mélange, la synthèse ou une réaction chimique quelconque.

21. Dispositif selon au moins l'une quelconque des revendications 1 à 20,
**caractérisé en ce que** le substrat de produits de réaction (9), la plaque de buse (5) ou le substrat de la chambre de réaction (6) fait l'objet d'un traitement chimique en surface (par exemple fonctionnalisé ou terminé).

22. Dispositif selon au moins l'une quelconque des revendications 1 à 21,
**caractérisé en ce que** le substrat de produits de réaction (9) est transparent, afin de mettre en oeuvre, entre autres, des appareils d'analyse comme par exemple des microscopes, des caméras CCD, des photodiodes, des phototransistors.

23. Dispositif selon au moins l'une quelconque des revendications 1 à 22,
**caractérisé en ce que** des composants électroniques comme des transistors, des diodes, des CCD, des photodiodes, des phototransistors, des résistances ou des électrodes pour la mesure d'impédance ou pour la voltamétrie cyclique sont intégrés dans le substrat de produits de réaction (9).

24. Dispositif selon au moins l'une quelconque des revendications 1 à 23,
**caractérisé en ce que** des composants électroniques comme des transistors, des diodes, des CCD, des photodiodes, des phototransistors, des résistances ou des électrodes pour la mesure d'impédance ou pour la voltamétrie cyclique sont intégrés dans la chambre de réaction (13) ou sur les substrats (6, 9).

25. Dispositif selon au moins l'une quelconque des revendications 1 à 24,
**caractérisé en ce que** la chambre de réaction (13) est conçue comme une cavité dans un substrat (6, 9).

26. Procédé pour la fabrication d'oligomères ou de séries d'oligomères utilisant le dispositif microélectromécanique selon l'une quelconque des revendications 1 à 25 par couplage progressif d'unités monomères à un matériau support solide, la protection intermédiaire s'effectuant par un ou plusieurs groupes de protection, qui sont transmis lors du couplage de l'unité monomère à la fin de la chaîne croissante, et qui sont enlevés avant la prochaine étape de couplage par dissociation du groupe de protection, et une étape de neutralisation supplémentaire est effectuée après la dissociation du groupe de protection, les réactifs utilisés pour la dissociation du groupe de protection et pour la neutralisation étant introduits dans des chambres de réaction (13) fermées les unes par rapport aux autres.

27. Procédé selon la revendication 26,
**caractérisé en ce que** des oligonucléotides sont utilisés comme oligomères.

28. Procédé selon la revendication 26,
**caractérisé en ce que** des oligoribonucléotides sont utilisés comme oligomères.

29. Procédé selon au moins l'une quelconque des revendications 26 à 28,
**caractérisé en ce que** des unités monomères abiologiques sont utilisées.

30. Procédé selon au moins l'une quelconque des revendications 26 à 29,
**caractérisé en ce que** la dissociation du groupe de protection s'effectue par un acide, par exemple de l'acide trichloracétique.

31. Procédé selon au moins l'une quelconque des revendications 26 à 30,
**caractérisé en ce que** la base utilisée pour la neutralisation est une base organique, par exemple de la collidine.

32. Procédé selon au moins l'une quelconque des revendications 26 à 31,
**caractérisé en ce que** l'injection des réactifs pour la dissociation des groupes de protection et pour la neutralisation s'effectue au moyen d'un élément de dosage de liquide (1, 4, 5, 7, 8) dans une microchambre de réaction (13), l'élément de dosage de liquide et la microchambre de réaction étant intégrés sur une puce.

33. Procédé selon au moins l'une quelconque des revendications 26 à 32,
**caractérisé en ce que** les réactifs utilisés pour la dissociation du groupe de protection et pour la neutralisation sont appliqués par un élément de dosage de liquide (1, 4, 5, 7, 8) sur des positions définies du matériau support.

34. Utilisation du dispositif selon au moins l'une quelconque des revendications 1 à 25 pour l'analyse de chaînes d'ADN ou d'oligomères.

35. Utilisation du dispositif selon au moins l'une quelconque des revendications 1 à 25 pour le mélange et la réaction de différents liquides, même corrosifs.

36. Utilisation du dispositif selon au moins l'une quelconque des revendications 1 à 25 pour le dosage et/ou le mélange de différents liquides sur un solide, par exemple pour l'analyse ou la réaction du solide.
